# EUROPEAN PATENT APPLICATION

(11) **EP 4 438 081 A1**
(43) Date of publication of application: **02.10.2024**
(21) Application number: 23164587.0
(22) Date of filing: 28.03.2023
(51) Int. Cl.: A61M 5/20, A61M 5/24, A61M 5/315, A61M 5/32

(54) **CODE AND CODE READER ARRANGEMENT FOR AN INJECTION DEVICE**

(71) Applicant: Ypsomed AG, 3401 Burgdorf (CH)
(72) Inventor: Scheurer, Simon, 3006 Bern (CH); Hostettler, Patrick, 3415 Hasle (CH); Allenspach, Marcel, 3400 Burgdorf (CH); Schüpbach, Simon, 3400 Burgdorf (CH); Büchi, Florian, 3074 Muri bei Bern (CH); Klötzli, Urs, 3414 Oberburg (CH)
(74) Representative: Kleiner, Stefan

(57) **Abstract**

The invention relates to an injection device (1) comprising a reservoir unit (10) and a drive unit (20) releasably attachable to the reservoir unit. The reservoir unit (10) comprises a reservoir (19), a reservoir holder (16) for holding the reservoir (19), a coupling member (17) and a machine-readable tag providing information and including a tag antenna (15). The drive unit (20) comprises a drive source for dispensing the liquid drug, a counter coupling member (23) adapted to engage the coupling member (17) to attach the drive unit (20) to the reservoir unit (10) in a predefined rotational position and a code reader for reading the tag information and including a reader antenna (25). In an attached state a center of the tag antenna (15) and a center of the reader antenna (25) are arranged to each other at an angle between 85° to 95° around the longitudinal axis.

## Description

### FIELD OF THE INVENTION

The present invention relates to an injection device for dispensing a liquid drug from a reservoir, the injection device comprising a syringe unit and a drive unit releasably attachable to the syringe unit. The syringe unit comprises a reservoir, a reservoir holder and a machine-readable code providing information and including a tag antenna. The drive unit comprises a drive source for dispensing the liquid drug and a code reader for reading the code information from the syringe unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic unit or control unit embedded or integrated in the delivery device, or being part of an auxiliary or supplemental electronic module or add-on device detachably attached to the delivery device. The electronic unit monitors a drug delivery process, in order to proactively prevent false handling of the device and/or to keep track of the doses already applied, and generates data related to an instantaneous condition and/or use of the delivery device. Suitable sensors of the electronic unit readily detect a status or signal from any kind of indicating component of the delivery device, including user interface elements and actuators. A wireless communication unit of the electronic unit is provided to wirelessly communicate, specifically upload, drug information to a nearby mobile device, a dedicated medical gateway or a cloud server. The drug information includes at least a time stamp and the expelled dose, indicative of a time of a medication event and of a quantity of delivered drug. The drug information may be transmitted instantaneously, or stored in a memory unit connected to the processing unit, for later upload or batch transfer.

The electronic unit may be able to read information from a reservoir or reservoir unit. In particular reusable devices or semi-reusable devices that allow to replace a reservoir may comprise a sensor that may read drug related information from a reservoir tag or code. The information thus read may be processed and stored by the electronic unit in the delivery device or additionally uploaded by the communication unit.

US 10804971 B2 discloses a reusable autoinjector comprising a syringe unit and a drive unit attachable to the syringe unit in two different rotational positions. The syringe unit comprises a housing adapted to hold a syringe with liquid drug. On an outside of the housing a RFID tag or an NFC tag is arranged. The drive unit includes two tag reader antennas adapted to read the tag of the syringe unit in either rotational position. If there are more than two possible attachment positions then there may be provided more than two tag readers antennas to be able to read the tag from the syringe unit by the tag reader of the drive unit in all possible attachment positions.

WO22013707 A1 discloses an electronic injection device with a replaceable medicament cartridge. A data tag is directly attached to an outside of a cylindrical cartridge barrel. The medicament cartridge can be inserted into a pivot mount element at any rotational orientation and thus the orientation of the data tag formed on the cartridge barrel is not predetermined. The electronic injector includes thus an annularly shaped electromagnetic detector enabling electromagnetic field lines of the electromagnetic detector to reach the data tag and vice-versa, irrespectively of the rotational orientation of the data tag.

US 2018/0236181 A1 discloses an electronic autoinjector adapted to receive a replaceable syringe. On an outside of a cylindrical syringe barrel a RFID tag is attached which can be read by a tag sensor. Three sensor coils are circumferentially arranged at an angle of 90° to each other in order to be able to read an electromagnetic field emitted from the tag and the electromagnetic field emitted from the sensor

### DESCRIPTION OF THE INVENTION

It is an objection to provide a simple and cost-saving data transfer interface design between a reservoir unit and a drive unit of an injection device.

This objective is achieved by an injection device according to the independent claim. Preferred embodiments are evident from the dependent claims.

The invention relates to an injection device for dispensing a liquid drug from a reservoir. The injection device comprises a reservoir unit and a drive unit releasably attachable to the reservoir unit. The reservoir unit comprises
- a reservoir or container, preferably a prefilled syringe with an injection needle, adapted to contain the liquid drug and defining a longitudinal axis;
- a reservoir holder for non-movably holding the reservoir;
- a coupling member, preferably arranged on the reservoir holder or on a reservoir housing and
- a machine-readable tag storing tag information and providing for tag information, wherein the code includes a tag antenna for a RF circuit. The tag may comprise a chip storing a machine-readable code, and a passive antenna connected to the chip.

The drive unit comprises
- a drive source or energy source for dispensing the liquid drug, preferably in a single dispensing stroke;
- a counter coupling member, preferably arranged on a drive housing or drive chassis. The counter coupling member is adapted to engage the coupling member to releasably attach the drive unit to the reservoir unit in a first predefined rotational position around the longitudinal axis and
- a code reader for reading the code information from the attached reservoir unit, wherein the code reader includes a reader antenna for an RF circuit.

In an attached state when the reservoir unit is attached to the drive unit a center or middle portion of the tag antenna and a center or middle portion of the reader antenna are arranged at an angle between 85° and 95°, preferably at an angle of about 90° to each other around the longitudinal axis. In a preferred embodiment there is exclusively a rotational offset between the tag antenna and the reader antenna and there is preferably no axial offset between the antennas. In case of predominantly planar antenna geometries, the two antenna planes intersect at an angle of at least approximately 90°.

According to the invention a center of the tag antenna of the reservoir unit and a center of the reader antenna of the code reader of the drive unit are arranged to each other in an angle between 85° and 95°, preferably at an angle between 87° and 93°, and more preferably about 90° around the longitudinal axis (seen in a plane perpendicular to the longitudinal axis). That means contrary to prior art approaches the tag antenna is not positioned vis-a-vis or in the same rotational position as the tag reader antenna when the reservoir unit is attached to the drive unit.

The code reader is configured to provide or generate an electromagnetic field enabling a data transfer between the tag antenna and the reader antenna. The data may be transferred according to a wireless technology such as radio-frequency identification (RFID) or a wireless standard such as near-field communication (NFC), Bluetooth, BLE or the like. It was found that the NFC Type 5 including tags according to ISO/IEC 15693 is particularly beneficial as data can be transferred wirelessly over a longer distance between the tag antenna and the reader antenna compared to other wireless standards.

The arrangement according to the invention offers the advantage that one single reader antenna is sufficient to cover at least two different rotational positions of the reservoir unit relative to the drive unit. That means as the tag antenna and the reader antenna are positioned at an angle of about 90° to each other the tag reader can read the tag information if the reservoir unit is attached to the drive unit in a first rotational position (for example 0°) and the tag reader can read the information of the reservoir unit attached in a different second rotational position (for example 180° rotated to the first position) relative to the drive unit. In either position the center of the tag antenna and the center of the reader antenna are arranged about 90° to each other.

Contrary to known tag and tag reader arrangements where several reader antennas at different places are required or one large circumferentially extending reader antenna is used to cover different rotational positions of the tag the arrangement according to the invention allows to use only one reader antenna covering all possible rotational positions of the tag of the reservoir unit. This reduces complexity and saves costs. Besides that, potential interferences of two or more reader antennas can be avoided.

The injection device includes the reservoir unit and the drive unit. The reservoir unit is not operational without the drive unit and vice versa. To prepare the injection device for an injection the user has to attach or to connect a reservoir unit to the drive unit. The drive unit may include drive means adapted to dispense the liquid drug from the reservoir hold inside the reservoir unit when the reservoir unit is attached to the drive unit.

The injection device is preferably a reusable or semi-reusable (or semi-disposable) injection pen, or semi-reusable autoinjector. The reservoir unit is preferably a disposable unit which is discarded after an injection or if the reservoir is empty. Accordingly, the drive unit is preferably a reusable part including a dispensing mechanism which can be reused for several injections and which can be attached to different reservoir units.

The reservoir unit houses the reservoir with the liquid drug. The reservoir may be a cartridge, a carpule or a syringe. The syringe is preferably a prefilled syringe including a syringe barrel and a needle or cannula that is permanently attached to a distal end of the syringe barrel. The syringe barrel is sealed at the opposing proximal end by a plunger or piston.

The drive unit preferably includes a housing that accommodates the drive source. The latter may include a dispensing member for example, a plunger rod, a drive sleeve, a clutch sleeve or any member movable relative to the reservoir during a dose dispensing operation of the injection device. The drive unit includes further a drive source in form of an automatic drive or a manual drive. Examples for an automatic drive are an electric motor or a releasable elastic element (for example a pre-tensioned spring) to drive the dispensing member to dispense the liquid drug form the reservoir. A manual drive may be dispensing button that has to be pressed by the user to move the dispensing member in the dispensing direction.

The reader antenna is a single element or circuit that provides for an electromagnetic field at a certain place on the drive unit. That means the reader antenna is position in one particular position on the drive unit and does not require several reader antennas distributed on different locations on the drive unit.

The tag antenna is preferably arranged in or onto a needle cover member, the reservoir holder, a housing or any other member of the reservoir unit or the tag antenna may be sandwiched in-between the reservoir and the reservoir holder or in-between the reservoir holder and a needle cover member of the reservoir unit. Alternatively, the tag antenna may be integrated in a component or in a label.

The read tag information from the tag may be, for example, stored in a storage module of the injection device, displayed to the user and/or wirelessly transmitted to an external receiver such as a cloud server, a HCP computer or healthcare facilities. The tag and the tag reader as described above may be part of an enhanced therapy concept including cloud services and remote user guidance. Namely, the read tag information from the tag may be transmitted from the injection device to a cloud server. The user, device and/or the drug information may be verified and compared with an external user-specific therapy plan or by a health care practitioner (HCP). Depending on the verification the user may be informed via display on the injection device and/or via user device.

The tag information may include, for example, information about the drug, a dose to be dispensed, a warm-up period of the drug, an expiry date or a drug identification number.

The coupling member and the counter coupling member are adapted to engage each other such that the reservoir unit and the drive unit are releasably connected to each other in one or more predefined rotational positions. The coupling member and the counter coupling member may allow the reservoir unit to be attached to the drive unit in just one rotational orientation. Alternatively, the reservoir unit and/or the drive unit may include a plurality of coupling members and counter coupling members allowing the reservoir unit to be attached to the drive unit in a plurality of rotational orientations to each other. In any case, in the attached state the a center of the tag antenna is positioned relative to the a center of the reader antenna at an angle between 85° and 95° seen in a plane perpendicular to the longitudinal axis. That means the tag antenna and the reader antenna are offset from each other at an angle of about 90°.

There is a comparable inductive coupling coefficient between the antennas irrespective of the orientation of the reservoir unit relative to the drive unit, albeit the inductive coupling coefficient is reduced with respect to the case of zero offset between the tag antenna and the reader antenna.

Examples for the coupling member and the counter coupling member are a spline and a nut, an external thread and a corresponding internal thread, a protrusion and a recess, a snap element and a cam or lobe adapted to engage the snap element.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the coupling member of the reservoir unit is one of a protrusion and a recess and the counter coupling member of the drive unit is the other of the protrusion and the recess. This allows for a reliable attachment as the protrusion can be inserted into the recess during the attachment process. The reservoir unit includes preferably one protrusion or two protrusions and the drive unit includes preferably two or more recesses allowing the reservoir unit to be connected to the drive unit in at least two different rotational orientations.

The drive unit includes preferably two counter coupling members oppositely arranged to each other such that the reservoir unit can be attached to the drive unit either in the first or in a second rotational orientation around the longitudinal axis. Preferably, the reservoir unit is in the second rotational orientation offset from the first orientation by an angle of at least approximately 180°.

That means the reservoir unit is attachable for example in a first orientation of 0° and in a second orientation at an angle of 180° relative to the first orientation. In either positions the center of the tag antenna and the center of the reader antenna are positioned at an angle between 85° and 95° (or about 90°) to each other around the longitudinal axis (seen in a plane perpendicular to the longitudinal axis).

Preferably, the tag antenna is arranged on the reservoir holder, on a housing or on a needle cover sleeve of the reservoir unit, preferably arranged on an outer surface of the reservoir holder, the housing or of the needle cover sleeve.

Alternatively, the tag antenna may be integrated in a wall of the reservoir holder, the housing or the needle cover sleeve, for example, covered or enclosed by injection molded polymers.

In a preferred embodiment the tag antenna is arranged circumferentially around an opening of a needle cover sleeve of the reservoir unit or around an opening of a reservoir holder or of a housing of the reservoir unit. The opening may be a through hole in a side wall allowing, for example, a release mechanism to engage a counter element in the drive unit if the reservoir unit is attached to the drive unit. Furthermore, the opening may be a viewing window for the user to view a fill level of the liquid drug contained in the reservoir.

The tag antenna located around the opening provides for a space-saving design and an unobtrusive placement.

In a further preferred embodiment the code is provided or integrated in a label affixed to an outer surface of the needle cover sleeve, the housing or the reservoir holder of the reservoir unit. That allows to hide the tag antenna inside or under the label which labels the drug and/or the reservoir unit. That means the tag antenna is protected from external influences such that the antenna cannot be rub off unintentionally.

The needle cover sleeve is preferably movable along the longitudinal axis between a covering position and a retracted position and guided by the reservoir holder. The cover sleeve may be implemented as sleeve or sleeve-shaped element adapted to cover or envelop an injection needle. The movement of the needle cover sleeve allows to switch between a safety state (covering position) in which the needle is covered and the needle does not protrude from a device housing and an injection state (retracted position) in which the needle is uncovered and exposes or protrudes from the housing.

In a preferred embodiment the needle cover sleeve is the outermost part of the reservoir unit and the reservoir holder is concentrically arranged inside the cover sleeve. Therefore, the cover sleeve is movable along the longitudinal axis outside and relative to the reservoir holder if the cover sleeve is not blocked or hold in the covering position. That means the reservoir unit does not comprise any outer housing or shell but only the movable cover sleeve. This provides for a simple design.

The needle cover sleeve may trigger an activation of the drive source. In particular, when the needle cover sleeve has reached a retracted or proximal end position the drive source may be activated to drive a drive member such as a plunger rod to dispense the liquid product.

Furthermore, the drive unit may comprise a code writer configured to write information to the tag in the attached state. That means the code reader is configured to transmit information from the drive unit (in particular from a controller or electronic unit) to the tag in the reservoir unit. By way of example, a current therapy plan status, a current device or environmental condition, a sensed fill level of the drug, a dispensed dose, a warm-up period or a time stamp can be written to the tag. This may help to identify or recognize the reservoir unit after a detachment and a re-attachment of the reservoir unit to the drive unit. For example, after attaching a used reservoir unit the drive unit may read out a fill level and the time of the last injection from a previously written or updated tag information.

The tag writer is preferably arranged in the same place as the code reader or the code writer is integrated in the code reader. That means the same antenna may be used to read information from the tag and to write information to the tag on the reservoir unit.

The information written by the tag writer of the drive unit to the tag of the reservoir unit comprises preferably an indication of a condition of use. That means after a dispensing injection data such as, for example, time and date and type of drug and/or therapy plan information are written to the tag. The updated tag may be read out at a later time. If, for example, the whole content of the reservoir was dispensed or if the reservoir must not used a second time the updated tag information may contain a warning or a stop command to avoid a second use of the reservoir unit.

The reservoir is preferably a prefilled syringe including a barrel and an injection needle permanently connected to the barrel and optionally covered by a needle shield in a shipping state. The prefilled syringe thus distinguishes from cartridges having a thread or connecting portion on the distal end where an injection needle has to be mounted to the cartridge prior injection.

The injection device with a prefilled syringe is thus an autoinjector and preferably a reusable autoinjector.

Alternatively, the reservoir is a cartridge and in this embodiment the injection device is a reusable injection pen including the replaceable cartridge.

The reservoir holder is adapted to hold the reservoir non-movable relative to the drive unit in the longitudinal direction. That means the reservoir or any element connected to the reservoir cannot move in the longitudinal direction. This is in contrast to devices that require the reservoir or a sub unit to move in distal direction to insert the injection needle into the injection site.

The drive source comprises preferably an electronic motor or a resettable or re-chargeable mechanical spring for emptying the entire volume of the reservoir in a single dispensing stroke. The drive source is accommodated in a drive unit housing or chassis holding or fixedly supporting the drive source.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a semi-reusable autoinjector including a reservoir unit and a drive unit according to the invention;
- Fig.2: depicts a perspective view of the drive unit;
- Fig.3a, 3b: depict schematical cross sections of a label including a tag of the reservoir unit and a tag reader of the drive unit and
- Fig. 4: depicts an exploded view of the reservoir unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

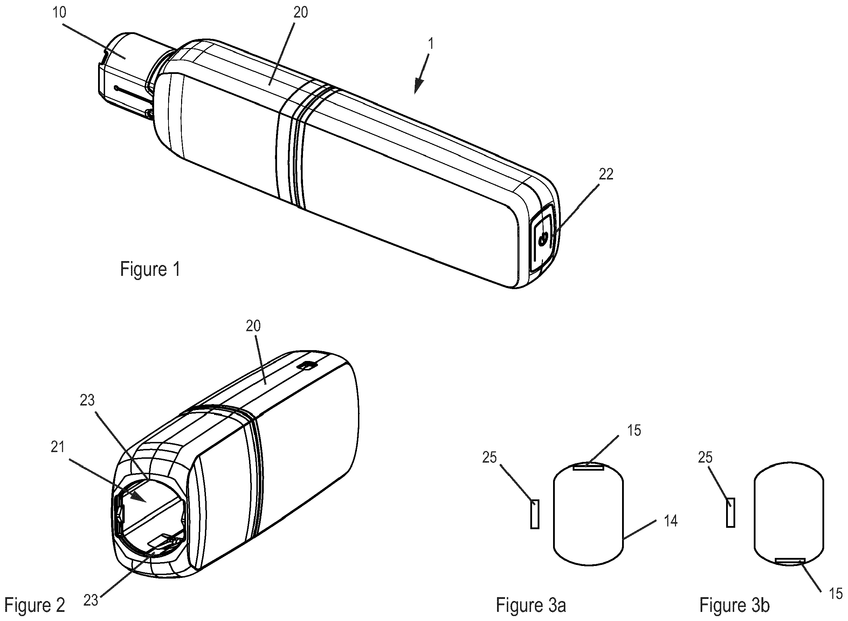

In the present description the term "distal" refers to the side where the injection needle is located. This is on the left hand side in the figures 1, 2 and 4. The term "proximal" refers to the opposite side or rear end of the device and is on the right hand side in figures 1, 2 and 4.

Figure 1 shows a perspective view of an injection device according to the invention in form of a semi-reusable autoinjector 1. The autoinjector 1 comprises a disposable reservoir unit in form of a syringe unit 10 and a reusable drive unit 20.

Figure 2 depicts a perspective view of the drive unit 20 without syringe unit. The drive unit 20 has an elongated housing defining a longitudinal axis and comprises a button 22 at its proximal end (see figure 1). On a distal side the drive unit 20 includes an opening or blind hole 21 (figure 2) adapted to accommodate the cylindrical-shaped syringe unit 10. On two opposite sides recesses or grooves 23 in the longitudinal direction are disposed on an inner surface of the hole 21. The grooves 23 are adapted to accommodate a coupling member in form of a protrusion 17 of the syringe unit 10. The grooves 23 thus act as counter coupling member for the coupling member of the syringe unit.

The drive unit 20 comprises further an electric motor for moving a plunger rod (not shown) in a dispensing direction to dispense the liquid drug form a syringe 19 inside the syringe unit 10. Furthermore, the drive unit 20 includes a NFC tag reader including a reader antenna 25 (figures 2a, 2b) adapted to read information from an NFC tag 15 of an attached syringe unit 10. The reader antenna 25 is located on one side and inside the opening 21 of the drive unit 20. The NFC tag reader is further configured to send data to a NFC tag and thus provides for a combination of a tag reader and a tag writer. The drive unit 20 further comprises a dispensing mechanism which is descripted in detail in the patent application EP22186392.1 which is hereby incorporated by reference.

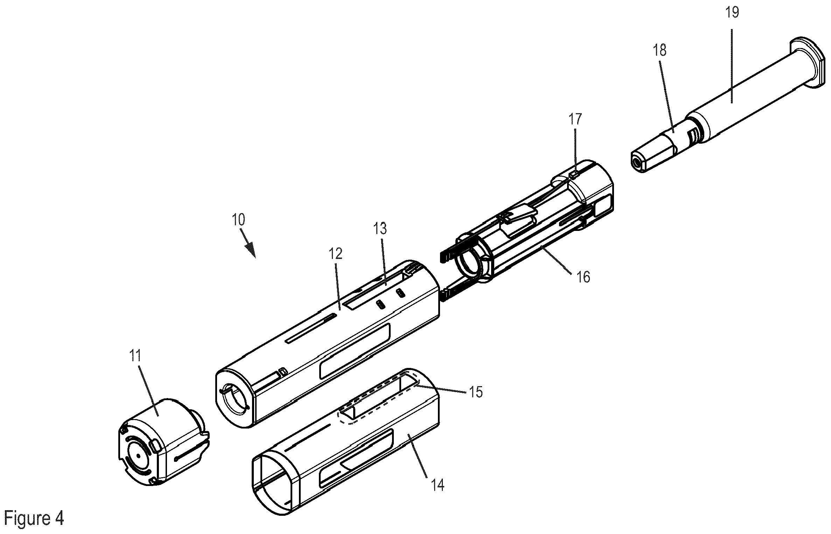

Figure 4 depicts an exploded view of the syringe unit 10. As shown in figure 4 the syringe unit 10 includes a syringe holder 16, a needle cover sleeve 12 coaxially arranged around the syringe holder 16 and movable relative to the syringe holder along the longitudinal axis, a label 14 wrapped around the needle cover sleeve 12, a cap 11 attached to a distal end and the prefilled syringe 19 comprising a liquid drug and a rigid needle shield 18 covering an injection needle (not shown).

The prefilled syringe 19 with a barrel made of glass is rotationally and axially fixed inside the sleeve-shaped syringe holder 16. For that purpose, the syringe holder 16 includes clamping elements (not shown) pressing on an outer surface of the syringe barrel and a distal shoulder of the syringe abuts a bearing surface inside the syringe holder 16.

The needle cover sleeve 12 is supported and guided by the syringe holder 16 and movable relative to the syringe holder between a covering position in which the needle is covered and a retracted position in which the needle is exposed. The needle cover sleeve 12 is concentrically arranged outside the syringe holder 16. That means the needle cover sleeve 12 is the outermost part of the syringe unit 10. The cover sleeve includes two oppositely located openings 13 that provide access to the protrusions 17 for attachment to the drive unit 20 as descripted below.

The label 14 affixed to the cover sleeve 12 comprises on a lateral side the NFC tag which includes a tag antenna 15 and which is integrated in the label 14. The NFC tag antenna 15 is circumferentially arranged around the opening 13 of the cover sleeve 12.

In order to attach the syringe unit 10 to the drive unit 20 the syringe unit is inserted with its proximal end into the distal opening 21 in the drive unit 20. The syringe holder 16 includes on two opposite sides the radially extending protrusions 17 that can be inserted into the grooves 23 inside the opening 21 of the drive unit. The protrusions 17 couple the syringe unit 10 to the drive unit 20. As the geometry of the two protrusions 17 and the two grooves 23 are symmetrical the syringe unit 10 can be inserted into the opening 21 in a first rotational orientation of 0° relative to the drive unit 20 and in a second rotational orientation which is 180° rotated compared to the first orientation.

Referring to figures 3a and 3b the arrangement of the NFC tag antenna 15 and the reader and writer antenna 25 is descripted in detail. Figures 3a and 3b depict a schematical and sectional view of the label 14 of the syringe unit 10 and the tag and writer antenna 25 of the drive unit 20. For the sake of clarity, other elements of the syringe unit and the drive unit are not shown. The cut for the sectional view is in a plane perpendicular to the longitudinal direction of the autoinjector 1.

As schematical depicted in figure 3a and 3b the reader and writer antenna 25 is not located vis-à-vis or opposite the NFC tag antenna 15 if the syringe unit is attached to the drive unit. In either attached position a center of the tag reader and writer antenna 25 of the drive unit is located at an angle of about 90° to a center of the tag antenna 15 of the syringe unit. Thus the rotational offset between the two antennas is about 90°. The tag reader and writer antenna 25 provides for an electromagnetic field strong enough to read the information of the offset located NFC tag antenna 15. A suitable communication standard includes but is not limited to the NFC Type 5 with tags according to ISO/IEC 15693.

As mentioned above the syringe unit 10 can be attached to the drive unit 20 in two different positions, namely 0° and 180° relative to the drive unit. Figure 3a shows the first possible orientation of the syringe unit with the tag antenna 15 on the upper side and figure 3b shows the second possible orientation with the tag antenna 15 on the lower side. That means in the second orientation (figure 3b) the syringe unit 10 is attached to the drive unit 180° rotated compared to the first orientation as shown in figure 3a.

In both, the first (0°) and second (180°) rotational orientation the distance between the tag antenna 15 and the reader antenna 25 is the same and the function remain unchanged. During dispensing the drive unit 20 can dispense the liquid drug from the reservoir in either rotational orientation of the syringe unit 10. In the same manner tag information can be read by the tag reader and writer and data can be written to the tag in either position.

The NFC tag provides information about the drug type, the syringe volume, the expiry date and a batch number. If prior injection a syringe unit 10 is attached to the drive unit 20 a controller in the drive unit is configured to read the tag information via its reader antenna 25. By way of example, the controller compares a read expiry data with the current date to verify whether the drug can be used or whether it already expired. In the latter case the controller informs the user accordingly or blocks the dispensing mechanism. Furthermore, the controller verifies whether the attached syringe unit is ready for use or if that syringe unit was already used and must not be used again. Additionally, the controller communicates via a communication module in the drive unit with a cloud server to obtain data of a user therapy plan. The server provides the injection information related to the identified drug and/or an identified user. The controller automatically sets a dose according to the received injection information and subsequently notifies the user about the time and the dose of next upcoming injections via display or user interface in the drive unit. Furthermore, the controller controls the tag writer to write a current fill level or use state information to the tag of the syringe unit.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: autoinjector
- 10: Syringe unit
- 11: Cap
- 12: Cover sleeve
- 13: opening
- 14: Label
- 15: NFC tag antenna
- 16: Syringe holder
- 17: Protrusion
- 18: Rigid needle sleeve
- 19: Syringe
- 20: Drive unit
- 21: Opening
- 22: button
- 23: groove
- 25: Tag reader antenna

## Claims

1. Injection device (1) for dispensing a liquid drug from a reservoir (19), the injection device comprising a reservoir unit (10) and a drive unit (20) releasably attachable to the reservoir unit, wherein the reservoir unit (10) comprises
the reservoir (19) defining a longitudinal axis;
a reservoir holder (16) for holding the reservoir (19);
a coupling member (17) and
a machine-readable tag storing tag information and including a tag antenna (15),
wherein the drive unit (20) comprises
a drive source for dispensing the liquid drug;
a counter coupling member (23) adapted to engage the coupling member (17) to attach the drive unit (20) to the reservoir unit (10) in a first predefined rotational position and
a code reader for reading the tag information and including a reader antenna (25),
**characterized in that**
in an attached state a center of the tag antenna (15) and a center of the reader antenna (25) are arranged to each other at an angle between 85° to 95° around the longitudinal axis.

2. Injection device (1) according to claim 1, wherein the coupling member (17) is one of a protrusion and a recess and the counter coupling member (23) is the other of the protrusion and the recess.

3. Injection device (1) according to claim 1 or 2, wherein the drive unit (20) includes two counter coupling members (23) oppositely arranged to each other such that the reservoir unit (10) can be attached to the drive unit (20) either in the first or in a second predefined rotational orientation around the longitudinal axis, preferably offset from the first orientation by an angle of at least approximately 180°.

4. Injection device (1) according to any of claims 1 to 3, wherein the tag antenna (15) is arranged on the reservoir holder (16), on a needle cover sleeve (12) of the reservoir unit (10) or on a housing of the reservoir unit (10).

5. Injection device (1) according to claim 4, wherein the reservoir holder (16), the needle cover sleeve (12) or the housing comprises an opening (13) and the tag antenna (15) is arranged circumferentially around the opening (13).

6. Injection device (1) according to claims 4 or 5, wherein the tag antenna (15) is integrated in a label (14) affixed to an outer surface of the needle cover sleeve (12).

7. Injection device (1) according to any of claims 4 to 6, wherein the needle cover sleeve (12) is movable between a covering position in which the needle is covered and a retracted position in which the needle is exposed and wherein the needle cover sleeve (12) is the outermost part of the reservoir unit and wherein the reservoir holder (16) is concentrically arranged inside the cover sleeve (12).

8. Injection device (1) according to any of claims 1 to 6, wherein the drive unit (20) further comprises a tag writer configured to write information to the tag in the attached state.

9. Injection device (1) according to claim 7, wherein the written information includes an indication of a condition of use of the syringe unit.

10. Injection device (1) according to any of claims 1 to 9, wherein the reservoir (19) is a prefilled syringe including a barrel and an injection needle permanently connected to the barrel and optionally covered by a needle shield (18) in a shipping state.

11. Injection device (1) according to any of claims 1 to 10, wherein the reservoir holder (16) is adapted to hold the reservoir (19) non-movable relative to the drive unit (20) in the longitudinal direction.

12. Injection device (1) according to any of claims 1 to 11, wherein the drive source comprises an electronic motor or a resettable or re-chargeable mechanical spring for emptying the entire volume of the reservoir in a single dispensing stroke.
